# EUROPEAN PATENT APPLICATION

(11) **EP 0 803 192 A1**
(43) Date of publication of application: **29.10.1997**
(21) Application number: 97500078.7
(22) Date of filing: 24.04.1997
(51) Int. Cl.: A01N 37/06, A01N 25/04, A61K 7/06, A61K 7/48

(54) **Antimycotic ectoparasiticidal product for external use**

(30) Priority: 24.04.1996 AR 33626296
(71) Applicant: Nunez, Omar Cristian, 1048 Buenos Aires (AR); Iannantuono, Rubén Fernando, 1048 Buenos Aires (AR); Katz, Noa Vera, 1048 Buenos Aires (AR); Katz, Esteban Miguel, 1048 Buenos Aires (AR)
(72) Inventor: Katz, Jaime, 1048-Buenos Aires (AR); Fernandez, Carbajales Juana, 1048-Buenos Aires (AR)
(74) Representative: Fernandez Lerroux, Aurelio

(57) **Abstract**

Antimycotic ectoparasiticidal product for external use consisting of the presentation of a known active agent, copper oleate, in a colloidal watery dispersion.

## Description

The present invention relates to an antimycotic ectoparasiticidal product for external use, applicable to both human and veterinary medicine. To be specific the present invention consists of a colloidal watery dispersion of copper oleate.

Ectoparasiticides are drugs which are used to treat skin infections caused by animal parasites. For human use, these drugs are principally insecticides and acaricides. Drugs currently available, used for the aforementioned purpose are:

### a) Lindane

Lindane or gammabenzene hexachloride has both an insecticidal and acaricidal effect and is used in the treatment of scabies. It makes up one percent of the ingredients in creams, lotions or shampoos. It is applied in fine layers from de neck down and must be left for a variable period (from 4 to 8 or 12 hours) depending on the patient's age and on the bodily affected area; afterwards a second layer must be applied on the fifth day and another on the tenth day, following the growth cycle of the parasite's eggs.

Lindane is used to treat all bodily areas where pediculosis is found. In general a single application of lindane of 1% in shampoo, lotion or cream for 4-8 or 12 hours (in accordance with the patient's age) is sufficient to eliminate the parasite since it is parasiticide and niticide.

Unfortunately lindane has a fast transcutaneous absorption level and a high liposolubility which provokes it to be collected in the fat tissue. It is inductor of the liver's oxidase system of mixed function, it breaks down slowly and remains in the body for a long time. It affects the presynaptic nervous terminals in central nervous system, increasing the release of many neurotransmitters, causing symptoms such as shivering, ataxia, convulsions and coma. Its carcinogenetic potential is doubtful in humans but it produces hepatomes in mice.

### b) Pyrethrins.

These are natural substances obtained from the pyrethrum plant (chrisanthemus circerapiaefolium). The pyrethroids are synthetic by-products whose main exponents are permethrin and decamethrin. They are of moderate efficiency since, although parasiticides, they do not eliminate nits.

They are available in the form of shampoo, lotion and cream rinse. They are applied to the scalp for a variable period of between 10 minutes and 8 hours ( according to the patient's age), and later the adherent nits are combed out.

Permethrin acts on the membrane of the nervous cell of the parasite, altering the current of the sodium channel which regulates polarization of the membrane. This causes an overdue re-polarization and the consequent paralysis.

Decamethrin shares the same properties as permethrin.

Pyrethrins and, in a bigger proportion, the pyrethroids have noticeable allergenic properties (contact dermatitis and respiratory allergies)

### c) Malathion

This is an organophosphorate insecticide, irreversible anticholinesterasic. It is highly efficient, but it is absorbed by the skin and can produce a severe muscarinic intoxication if the instructions for use are not strictly followed, for which it is not recommendable for use.

### d) Benzyl benzoate

Especially useful for the treatment of scabies and it is also used in the treatment of pediculosis. It is an irritant and frequently causes contact dermatitis and for this reason its use is not advisable.

### e) Diethyltoluamide

The most used insect repellent. Its absorption by the skin is estimated to be between 10% and 15%. It produces allergic reactions and, in children, toxic encephalopathy, erythema and cutaneous ulcerations.

### f) Copper

Copper is a trace element which forms part in many of the most important chemical reactions of the human organism, among which are those related to erythropoyesis.

The daily requirements of copper are around 2,5 mg and this is easily covered by the diet. The increase in contribution is eliminated by the organism by means of a decrease in its gastrointestinal absorption by a complex homeostatic regulator mechanism.

Copper used in the form of salts (oleate or sulphate) has antiseptic (sulphate), fungicidal (sulphate and oleate) and parasiticidal (oleato) properties.

Copper salts possess antiseptic properties which are not very powerful, it is more a question of bacteriostatic effects when it is used in its normal concentration.

The fungicidal property of the copper sulphate is used especially to destroy parasitic fungi of plants and as regards the mechanism of the action, it is accepted that the copper cations precipitate the proteins, and the local antiseptic effects are due to this, depending on the concentrations used.

The ectoparasiticidal property is not fully understood but it is thought that the copper could act as a neurotoxic agent on parasites. Around 30% - 40% of the copper consumed is absorbed by the stomach and duodenum which is followed by rapid transport to the liver, united in lax form to the albumin. These complexes are separated in the membrane of the hepatocytes and the pure copper is transferred to the inside of the cells where it mixes with the apoceruloplasmin in which form it is excreted towards the serum.

The ceruloplasmin takes between 90% and 95% of the plasmatic copper and is normally recycled in the liver, where it is degraded in the lysosomes freeing the copper which is then excreted towards the bile. The rest of the plasmatic copper is weakly united to the albumin and in part is excreted towards the bile. The quantity excreted by the kidney is minuscule compared with the excreted by the biliary ducts.

The swallowing of excessive doses, for example 10 or more grammes of copper sulphate causes symptoms derived form its local irritant action in the gastrointestinal tract; it produces nauseas, vomiting, colic and diarrhoea which can lead to collapse and shock.

The formulations of ectoparasiticides that contain copper, known to date, are mixtures of inflammable hydrocarbons, containing a copper salt of a fatty acid, dissolved in organic solvents.

One obvious difficulty of these products is the high risk of intoxication, since its formulation favours cutaneous absorption and remains in the tissue where it is absorbed. On the other hand, due to its high irritant action any contact of this type of product with the eyes, mucous membranes and open wounds must be avoided. Another adverse condition is the fact that there should be no contact with fire, due to its inflammability. As for the form of use, 3 or 4 spoonfuls of the mixture should be applied to the affected areas, left overnight and then washed off with abundant soap and water.

In the case of any of the aforementioned problems occurring through the use of the product, the objective of this invention is to provide a product with efficient external ectoparasiticidal and pediculicidal action, exempt from the risk of toxicity in human beings or in animals exposed to it, while offering more favourable use conditions.

The present invention provides a solution to reach the set goal, constituting a creative idea, consisting of a novel formulation of copper oleate, which preserves its insecticidal and fungicidal properties, associated at the same time with a practically non cutaneous absorption. The aforesaid formulation, upon which the present patent of invention is based, represents a creative response to the specific problems created in relation to the means and conditions in which this type of product is used and is fundamentally different to those currently used. Therefore, its industrial production will cover a current need in the market and presents a productive novelty.

The present invention is illustrated by means of the following examples which should not be taken as restrictions of the same.

### EXAMPLE 1: Shampoo

| Cuali-quantitative formulation expressed in centesimal form: | |
|---|---|
| **CHEMICAL NAME** | **PERCENTAGE BY VOLUME** |
| Sodium laurylethersulfate 25 % | 30,00 g |
| Diethanolamide of fatty coco acids | 2,50 g |
| Copper oleate | 1,00 g |
| Perfume | 0,25 g |
| Destilled water c.s.p. | 100,00 cc |

An additional advantage of this example is its use, which consists of applying it, leaving it for one minute to act, rinsing, and repeating the operation if necessary.

### EXAMPLE 2: Lotion

| Cuali-quantitative formulation expressed in content of each 100 cc: | |
|---|---|
| **CHEMICAL NAME** | **CONTENTS BY VOLUME** |
| Sodium laurylethersulfate 25 % | 10,00 g |
| Diethanolamide of fatty coco acids | 1,00 g |
| Copper oleate | 0,20 g |
| Polyvinylpyrrolidone | 0,50 g |
| Perfume | 0,10 g |
| Glycerine | 1,00 g |
| Water c.s.p. | 100,00 cc |

### EXAMPLE 3: Cream

| Cuali-quantitative formulation expressed in content of each 100 cc: | |
|---|---|
| **CHEMICAL NAME** | **CONTENTS (p/p)** |
| Stearic acid | 20,00 g |
| Glycerine | 40,00 g |
| Ammonia (26° Bé) | 3,00 cc |
| Copper oleate | 1,00 g |
| Perfume | 0,20 g |
| Water c.s.p. | 100,00 g |

An external ectoparasiticidal and pediculicidal product is therefore provided, in accordance with the present invention, which offers the following advantages and benefits, standing up to existing products with this aim.
1. It has a high ectoparasiticidal (especially on louse and acarus) and fungicidal property, in addition to excellent cosmetic properties.
2. It is easy to apply, whatever its final pharmaceutical form.
3. It does not irritate the skin and mucous membranes
4. It presents a very low risk of allergenic phenomena.
5. It can be used continually.
6. It is practically atoxic.
7. It can be used with all ages.
8. In the case of the shampoo and the lotion, they have a pleasant crystal emerald green appearance which favours the acceptance of the consumers.

To conclude, this invention is based on a new concept consisting of the presentation of a know active agent (copper oleate) in a colloidal watery dispersion.

Furthermore, it is undoubtable that this invention is not limited to the examples exactly described, but can undergo diverse changes and modifications, without moving away from the scope of this invention. Consequently, in making the external ectoparasiticide and fungicide described and illustrated, modifications can be introduced and/or improvements, all of which should be considered as variants of application comprised within the range of protection of this patent of invention, the range which is determined, fundamentally, by the claimed clauses which follow.

Having described and determined the nature and range of this invention and the manner in which it should be used, it is declared that what is claimed as an invention of exclusive rights is:

## Claims

1. An antimycotic ectoparasiticidal composition which includes copper oleate as an active agent, characterised by being vehiculised this copper oleate in the form of a colloidal watery dispersion.

2. An antimycotic ectoparasiticidal composition as claimed in point 1., presented in the form of shampoo, characterised by the proportion of the referred active agent is found to be comprised of between 0,1 and 2% in weight and because the referred colloidal watery dispersion is a compound of a watery solution of sodium laurylethersulfate 25 % in a proportion comprised of the range which goes form 20-40% in weight of the product, of diethanolamide of fatty coco acids, in a proportion comprised of the range which goes form 1-3% in weight, of perfume in a proportion comprised of between 0 and 1% in weight and of destilled water in a sufficient quantity to complete the 100% composition.

3. An antimycotic ectoparasiticidal composition as claimed in point 1., presented in the form of lotion, characterised because in each 100 cc of the product, the contents of the said active agent is in the range comprised of between 0,1 and 0.3 g.; and the referred colloidal watery dispersion is a compound of a watery solution of sodium laurylethersulfate 25 % in a quantity comprised of between 5-15g., of alkylamid in a quantity comprised between 0,5 and 1.5 g., of polyvinyl pyrrolidone in a quantity comprised of between 0,2 and 0,8 g., of glycerine in a quantity comprised of between o,5 and 1,5 g., of perfume in a quantity comprised of between 0 and 1g., and of destilled water in a sufficient quantity to complete the 100 cc of the composition.

4. An antimycotic ectoparasiticidal composition as claimed in point 1., presented in the form of cream, characterised because the contents of the said active agent in each 100 g. of the product is in the range comprised of between 0,1 and 1,5 g.; and the referred colloidal watery dispersion is a compound of stearic acid in a quantity comprised of between 15-25g., of glycerine in a quantity comprised of between 30 and 50 g., of ammonia (26° Bé), in a quantity comprised between 2 and 4 cc, of perfume in a quantity comprised of between 0 and 1g., and of destilled water in a sufficient quantity to complete the 100 g. of the composition.
